# EUROPEAN PATENT APPLICATION

(11) **EP 4 643 895 A1**
(43) Date of publication of application: **05.11.2025**
(21) Application number: 24210110.3
(22) Date of filing: 31.10.2024
(51) Int. Cl.: A61L 27/44, A61K 6/00

(54) **BIO-BASED BINDER FORMULATION APPLIED IN ORTHOTICS AND ORTHOPAEDIC DEVICES**

(30) Priority: 03.05.2024 PT 2024119452
(71) Applicant: Ptscience, Unipessoal, Lda, 2460-071 Alcobaça (PT)
(72) Inventor: BORREGO DA ENCARNAÇÃO, TELMA MARIA, 2460-521 ALCOBAÇA (PT); FERREIRA DOS SANTOS, FÁTIMA DA CONCEIÇÃO, 4650-029 AIÃO, FELGUEIRAS (PT); SINGH, POONAM, 3000-316 COIMBRA (PT); DOS SANTOS MATEUS, ARTUR JORGE, 2400-187 LEIRIA (PT)
(74) Representative: Moniz Pereira, Manuel

(57) **Abstract**

The present invention refers to a bio-based binder formulation/composition for application in orthotics and orthopaedic devices as a cushion for plasters and splints. The present invention provides a bio-based binder formulation/composition comprising a hydrocolloid component, water, an alcohol, a polyphenolic compound and iron compounds. Additives can be added to the bio-based binder, to enhance its properties in accordance with the final application.

## Description

### Field of the invention

The present invention falls within the scope of binders and refers, more particularly, to a bio-based binder formulation for application in orthotics and orthopaedic devices. The bio-based binder referred to in the present invention has the ability to be used alone, or with the ability to aggregate different materials, such as waste material (giving value to products that otherwise had no commercial value) being eco-friendly and sustainable.

### Summary of the invention

The object of this invention is to provide a bio-based binder formulation/composition for application in orthotics and orthopaedic devices as a cushion for plasters and splints.

The present invention provides a bio-based binder formulation/composition comprising a hydrocolloid component, water, an alcohol, a polyphenolic compound and iron compounds.

Additives can be added to the bio-based binder, to enhance its properties in accordance with the final application. Kaolin can be one of the added additives.

### Background of the invention

A binder agent, or simply binder is defined as a substance or material that is used to form a cohesive whole, chemically, mechanically, or even by adhesion or cohesion. These substances are present in liquid or powder form and are employed in order to give cohesiveness to otherwise loose components 1,2. They are present in the form of solvent sprays, emulsions, veils, and powder sprays being the choice of the binder, dependent on factors such as operational effectiveness, compatibility with the matrix, the process environmental control the performing technique, and the performance of the final product 3.

Binder agents can be divided according to their origin/source (natural/ synthetic polymers) but also based on their application: dry binders, added to the powder blend ( i.e. cellulose); or solution binders, dissolved in a solvent- water or alcohol are used in wet granulation processes (i.e. starch, sucrose, polyvinyl pyrrolidone, among other examples) ^{1,4}. Binders can be used with polyester, epoxy, and other types of liquid resins ³.

Natural binders are usually used in different fields (i.e. pharmaceutical, and food industries) because they are available, present low cost, low toxicity, and are environmentally friendly, being biodegradable ¹. Tragacanth is a natural binding agent in the form of gums and is used to bind tablets, incense, and pastel paints, among other applications. Candelilla is a wax applied as a binder in chewing gum and Gum arabic is a gum used in cosmetics, photography, and ceramics, among others ¹.

Synthetic binders are composed of oils, resins, and polymers and can present improved mechanical properties ¹. Also, they can impart other important functions such as being a toughening agent (with the addition of thermoplastics) ³.

Within the synthetic binders used for polyester and vinyl ester resins some examples are the isophthalic polyester resins, the UV-curable monomers, the unsaturated polyester resin, the VR-60, the Orgasol 1002 among others ³.

Binders (synthetic) for epoxy resins include non-catalyzed epoxy resins, catalyzed epoxy, liquid-catalyzed RTM epoxy, acrylonitrile-vinyl chloride copolymer, epoxy resins with thermoplastics, cured and uncured epoxies with multiwall carbon tubes, graphene oxide, among others ³. The components include acrylonitrile, vinyl chloride, fluorene epoxy resin, polyamides, polysulphones or phenoxies, graphene oxide ³.

According to the manufacturers, many binders are universal materials that can be used with most types of resins, being that it is recommended that the chosen binder has the same nature as the matrix where it is going to be impregnated in ³. But some binders can also be applied with different resins: epoxy-based binders can be used for matrices of bismaleimide and benzoxazine and a binder based on bisphenol A epoxy resin (curing agent-dicyandiamide; thickener- polyacrylamide; catalyst- imidazole) can be compatible with matrices such as polyester, vinyl ester, phenolic thermoplastics, and urethane polymers ³.

Binder systems are often used in the production of composite materials with recurrence to modern technologies (cheap, quick, complex-profile products) allowing for the development of products such as cars, aircraft, and devices for household purposes among many other applications ³.

Binders based on epoxy and acrylic resins are the most used, presenting advantages as being cheap, allowing for regulation of their chemical and physical properties, and producing good quality products. Binders based on polycarbonate, polyamide, polyurethane and many others are also used ³.

In the wood industry, binders have been extensively applied and include phenol-formaldehyde, phenol resorcinol formaldehyde, and urea formaldehyde due to their advantageous properties (better performance than natural binder, waterproof and water resistance) ^{2,4}. Phenolic resins and isocyanates are also used ^{4,5}. An approximate volume of 11 million tons/year of ureaformaldehyde adhesives is used around the globe being this the most widespread and low-cost binder ². Polyethylene, polypropylene, polyvinyl chloride, and other copolymers are used as wood composite binders ².

Formaldehyde (carcinogenic) can be released from the wood panels and cause eye, throat, and nose inflammation, an asthma-like-respiratory allergy among other negative effects on humans. In nature, formaldehyde decomposes in the air forming carbon monoxide and formic acid. The toxicity of formaldehyde towards animals includes reproductive problems lower fertility and a decrease in the lifespan of the exposed organisms (compromising the next generation and ecosystem equilibrium and services). In 2008, the Environmental Protection Agency (EPA) declared formaldehyde a carcinogenic material ⁴.

Polyurethane resins are highly inflammable and protective equipment is needed when handling them due to the release of free isocyanate groups in contact with human skin, being that the catalysts and stabilizers used are strong irritants and are toxic, even in small quantities ⁶.

Synthetic binders are made of fossil-based derivatives, with huge concerns for the environment: non-renewable resources, increase in carbon footprint, greenhouse gas emissions, toxicity towards humans and ecosystems, recyclability problems (disposal in landfills or incineration-release of toxic components).

Given the negative aspects of synthetic binders, such as the environmental impacts, and the increase in plant and animal waste, an interest in new binders that are biologically based has been increasing over the years ^{3,4}.

Different bio-based binders have been described in the literature, as mentioned previously. Examples of binder compositions can be found in patent documents such as CA3023970A1 and CA3062961A1. However, no document describes a bio-based binder composition such as that of the present invention.

The present invention relates to a bio-based binder formulation that is non-toxic, biodegradable, environmentally friendly and exhibits good properties, while having low costs and the ability to be used in a variety of products.

### Advantages of the invention

The present invention relates to a bio-based binder formulation that is non-toxic, biodegradable, environmentally friendly and exhibits good properties: the glass transition temperature happens at 70 °C and a melting point of 140°C; the degradation of the material starts at 80 °C and it is mainly constituted of carbon, oxygen and nitrogen. The hardness of the material is adaptable/tuneable in accordance with patient needs. At the same time, the bio-based binder formulation has low costs and the ability to be used in a variety of products. It can be applied in different solutions for different parts of the body (different sizes and shapes are possible) and it helps with relieving pressure and providing comfort to the patient.

According to the present invention, the bio-based binder is safe and can be used as a natural binder for natural fibres, such as, but not limited to, cork. It can be used in orthotics and orthopaedic devices as a cushion for plasters and splints, but also on insoles, offloading pads and cushions or many other related applications were avoiding skin maceration and pressure sores in needed.

The bio-based binder can be applied in different solutions for different parts of the body (different sizes and shapes are possible) and help relieve pressure and provide comfort for the patient.

The bio-based binder formulation can be used in insoles, unloading pads and cushions or many other applications.

In addition, the bio-based binder of the invention has flame retardant properties.

### Brief description of the figures

These and other features can be easily understood from the attached drawings, which should be considered as mere examples and in no way restrictive of the scope of the invention.
Figure 1 shows the Attenuated Total Reflectance - Fourier Transform Infrared Spectroscopy (ATR-FTIR) spectrum of the bio-based binder of the present invention, in the frequency regions of 4000-600 cm⁻¹.
Figure 2 shows the Differential Scanning Calorimetry (DSC) graphical results for bio-binder of the present invention with iron (III) chloride in its formulation, which confers it a black colour.
Figure 3 shows the Thermogravimetric analysis for bio-binder of the present invention with iron (III) chloride in its formulation, which confers it a black colour and for bio-binder of the present invention with iron (III) oxide, which confers it a red colour.
Figure 4 show the Scanning Electron Microscopy (SEM) images of the bio-binder of the present invention with iron (III) oxide in its formulation.

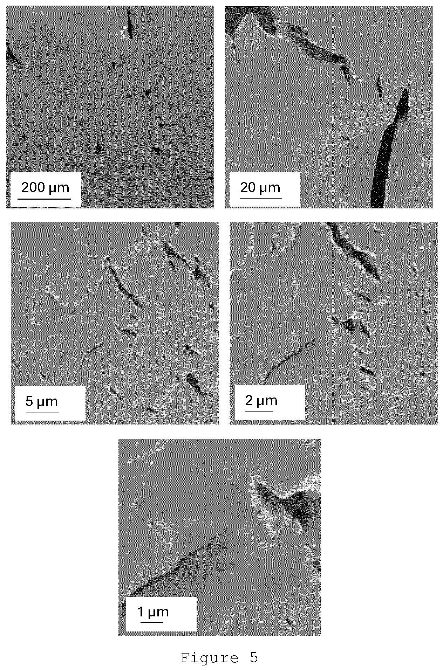

Figure 5 show the Scanning Electron Microscopy (SEM) images of the bio-binder of the present invention with iron (III) chloride in its formulation.

### Detailed description of the invention

The object of the present invention is a bio-based binder composition/formulation comprising a hydrocolloid component, water, an alcohol, a polyphenolic compound, and iron compounds.

Additives can be added to the bio-based binder, to enhance its properties in accordance with the final application. Kaolin can be one of the added additives.

The bio-based binder itself can be modified chemically, using techniques such as plasma treatment techniques.

Fibers to be bounded by the material of the invention can be chemically or naturally modified. Different types of fibres can be used, such as cork, wood fibres, and luffa cylindrical, among others. Modifications can be methylation, sulfonation, acetylation, and nitration among other modifications.

### Hydrocolloid component

Hydrocolloids can be from animal or vegetal sources (also from microbial or synthetic origin) being partially or completely soluble in water and swellable and/or dispersible in water. Their characteristics and properties depend on the source and the production technology/techniques used. They present very good gel-forming, foaming, and water-biding properties due to their structure and ions in the molecular chain (that gives them great water-binding capacity and high solubility). Along with the excellent water-binding properties, the ability to form a network of proteins are the most valuable properties of hydrocolloids.

The hydrocolloid component is selected, namely, but not exclusively, among: agar agar, vegetal gelatine, pectin, alginate, cellulose, cellulose derivates, xanthan gum, carrageenan (helps to enhance texture while improving water absorption), preferably gelatine.

The content of the hydrocolloid component should be up to 20 wt% of the bio-based binder, preferably between 15 and 20%, even more preferably between 17 and 19%.

### Water component

The addition of water in the present invention allows for the development of hydrogen bonds between the water molecules and the hydroxyl groups of glycerol leading to the increase in the content of the water bond in the invention material. Depending on the proportions of the elements in the invention, including water, the mechanical properties can be improved. With the addition of water, viscosity can be better controlled (promoting better adhesion to the substrate and a good coating and impregnation process). Being water the universal solvent, it can help with the dispersion of components of the invention (homogeneity) and also be a carrier promoting good impregnation in the materials being treated. By using water, other components, such as volatile organic components, can be reduced making the developed binder more friendly towards the environment.

The content of water component should be up to 50 wt% of the bio-based binder, preferably between 30 and 48%, more preferably between 40 and 48%.

### Alcohol component

The binder composition of the present invention comprises an alcohol component selected, namely, but not exclusively, among: a polyhydric alcohol (C₃H₈O₃), being a polyol comprising 3 hydroxyl groups, such as, but not limited to, ethylene glycol and propylene glycol. The preferred alcohol component for the present invention is glycerol.

Glycerol combined with glycerides oils and fats and/or as lipids in the intracellular spaces occur naturally in plants and animals. This compound is regarded as safe as a food additive, being authorized in the UE. Glycerol also has found applications in the cosmetic industry, the pharmaceutical industry, and many other applications (intermediate and monomer, namely, but not exclusively, in resins, polyols and polyurethanes, paints, varnishes, adhesives, binding agents).

In addition, glycerol can be used as a plasticizer in developed binders to reduce vitrification effects and improve mechanical properties. Hydrogen bonds between the three hydroxyl groups of glycerol (non-solvent for gelatine) and water molecules are formed leading to an increased content of bound water in the material of the invention. The addition of glycerol promotes strong bonds that reduce the ice crystals formation and imparts anti-freezing properties (a solution of glycerol/water is used as nontoxic antifreeze). In addition to gelatine, glycerol improves flexibility, elasticity, and, by decreasing interactions between adjacent gelatine chains, it also improves moisture sensitivity.

At the same time, the mechanism of gelation is not affected by the addition of glycerol.

The content of alcohol component should be up to 30 wt% of the bio-based binder, preferably between 10 and 30%, more preferably between 25 and 30%.

### Polyphenolic compound

The polyphenolic component suitable for the present binder comprises preferably a tannic acid. Tannic acid is a cross-linkage agent. Tannic acid presents hydroxyl groups that could form hydrogen bonds with gelatine chains. Tannic acid is a natural amphiphilic polymer presenting good water solubility and containing a large number of hydrophilic phenolic hydroxyls in its hydrophobic backbone. Tannic acid stability is increased when binding with proteins rich in proline (such as gelatine) due to the interactions between the hydrophobic regions of proteins and the aromatic ring of tannins. In addition, tannic acid can induce cross-linkage within the gelatine structure increasing the physical properties of the material obtained.

The content of the polyphenolic component should be up to 10 wt% of the bio-based binder, preferably between 5 and 10%, even more preferably between 5 and 9%.

### Iron compounds

The binder composition of the present invention comprises iron compounds selected, namely, but not exclusively, among iron chloride (III), iron sulphate, preferably iron oxide (III).

The formulation of the bio-based binder of the present invention, when it comprises iron (III) chloride in its formulation, is black in colour and when the formulation of the bio-based binder of the present invention comprises iron (III) oxide, it is red in colour.

The addition of iron has the effect of improving the properties Addit of the material. The water molecules present on the material of the invention can be replaced by iron ions, forming, by a strong action, a strong ion crosslink between molecular chains.

The content of the iron compounds should be up to 5 wt% of the bio-based binder, preferably between 1 and 5%, even more preferably between 3 and 5%.

### Additives compounds

Additives can be added to the bio-based binder to improve its properties according to the final application, in particular but not exclusively, kaolin, zinc dioxide, silicon dioxide and magnesium oxide.

Kaolin is an inexpensive additive that can be added to material to reduce its costs. The addition of kaolin to a formulation can improve processing, storage, and application having a hydrophilic nature. The addition of kaolin can enhance the appearance (sheen, and gloss), improve corrosion, and scrub resistance, and increase durability by being more weather resistant.

The content of the additive's compounds should be up to 5 wt% of the bio-based binder, preferably between 1 and 3%.

### Bio-based binder characterisation

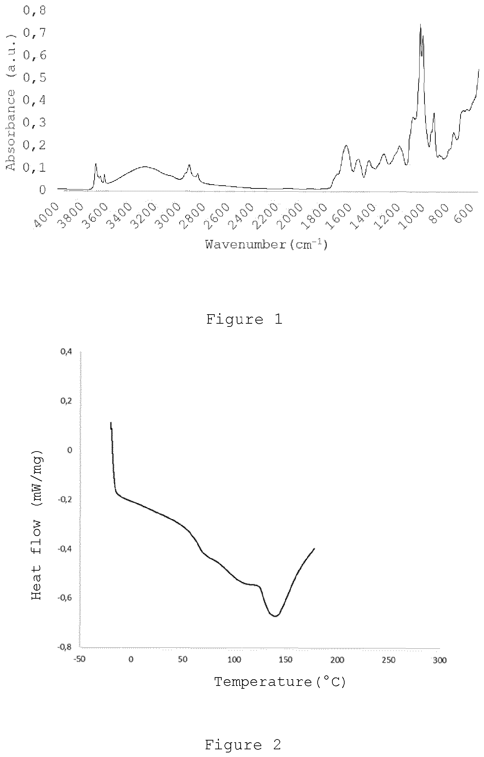

According to Figure 1, the peaks from the ATR-FTIR spectrum of the invention material could be assigned as follows: In the range of 3250-3300 cm⁻¹, a broad peak emerges, which is attributed to -NH and -OH stretching vibrations. This region is characteristic of the stretching vibration of hydroxyl groups in the gelatine and tannic acid compounds. The hydroxyl groups from glycerol further enhance this band. The peaks around 2900 and 2845 cm⁻¹ are due to the symmetric and asymmetric stretching vibration modes of CH and CH₂ of glycerol and gelatine. The discrete peak that emerges at 1717 cm⁻¹ is attributed to the presence of CO in the carbonyl group of tannic acid. At 1621 cm⁻¹, the peak indicates the stretching vibration of C=C, confirming the successful incorporation of the tannic acid into the formulation, whereas the peak at 1526 cm⁻¹ is due to the -NH and -CN groups belonging to the amino acid molecules of the gelatine, and the signal at 1312 cm⁻¹ is due to the C-N stretching vibrations of the gelatine molecule. Two intense peaks, at 1027 and 1006 cm⁻¹, correspond to the OH stretching vibration from glycerol. The peaks at 3683, 3644 and 3616 are due to the presence of kaolin.

According to Figure 2, the bio-based binder with iron (III) chloride, which is black in colour, has a glass transition temperature (T_{g}) of 70 °C and a melting point (Tm) of 140 °C.

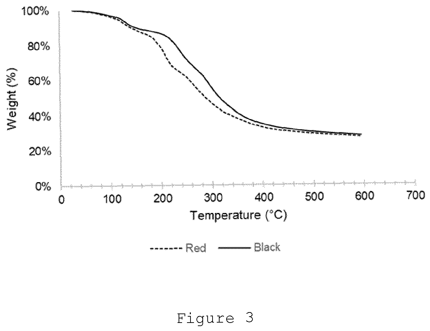

Figure 3 shows the Thermogravimetric analysis for bio-binder of the present invention with iron (III) chloride in its formulation, which confers it a black colour and for bio-binder of the present invention with iron (III) oxide, which confers it a red colour. From the graph present in Figure 3 it is possible to observe that both variations of the material (black and red colour) seem to present a similar thermogravimetric behavior indicating a similar degradation profile. When looking at the calculated values in Table 1, it is observed that the initial degradation temperature for both materials is similar (77,6 °C and 84,6 °C) but the temperature related to the maximum weight loss (Tₚ) differs for the red and black materials. For the red binder agent, the maximum rate of weight loss happens at 206,6 °C and for the black binder agent, the maximum rate of weight loss happens at 301,5 °C. In both teste materials, the material left in the end of the analysis (at 600 °C) is around 27 %. This indicates that a significant amount of the material is not degraded even at such high temperatures.

**TABLE 1**

| | **Red*** | **Black**** |
|---|---|---|
| T₂ (°C) | 77,6 | 84,6 |
| Tₚ (°C) | 206,6 | 301,5 |
| Material left (%) | 27,05 | 27,9 |

| | | |
|---|---|---|
| *Bio-based binder of the present invention, when it comprises iron (III) oxide in its formulation. **Bio-based binder of the present invention, when it comprises iron (III) chloride in its formulation. | | |

The SEM images of the bio-based binder of the present invention, when it comprises iron (III) oxide in its formulation (Figure 3) seem to show a surface with several roughness where the material does not seem to be very homogeneous. There seems to be some cracks and imperfections on the material surface possibly related to the constituent's mixture.

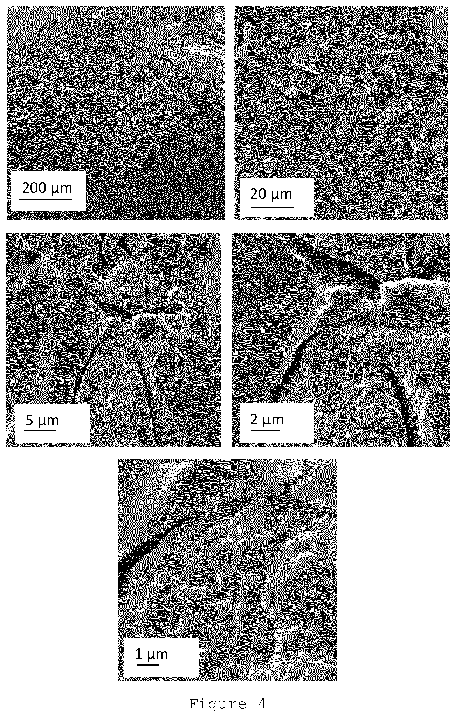

The SEM images of the bio-based binder of the present invention, when it comprises iron (III) chloride in its formulation (Figure 4) show a material with a flat surface with some cracks and imperfections. Even so, the material seems to be more homogenous than the bio-based binder of the present invention, when it comprises iron (III) oxide.

### Production method

The method to produce bio-based binder of the present invention comprises the following steps:
a) adding the dry components: hydrocolloid component, polyphenolic compound, kaolin and the iron compounds;
b) heat the mixture obtained in the previous step to between 70 and 100°C and allow the mixture to dissolve by stirring
c) mix the water component with the alcohol component;
d) add the water component and alcohol component to the mixture obtained in step b) and mix all the components so that the mixture is homogeneous;
e) the mixture obtained in the previous step will change colour, indicating that the reaction is taking place (to a greenish colour and then to a black material);
f) the mixture obtained in the previous stage separates into two phases: a liquid phase and a gel-like material;
g) the liquid phase is discarded;
h) a gel-like material is cooled and allowed to dry at room temperature for at least 24 hours.

Additionally, when the iron compound selected is iron chloride (III), kaolin is added to the formulation.

Additionally, when the iron compound selected is iron oxide (III), heating - cooling cycles are carried out, which comprise the following steps:
a) when gel-like material obtained in step h) has cooled, it is heated for between 10 and 15 minutes until it reaches the temperature of at least 70 °C;
b) a heated mixture is obtained and allowed to cool again for 30 and 45 minutes;
c) the mixture will start change consistency with the heating-cooling cycles (more viscous and harder). Repeat the heating- cooling steps (steps a) and b)), until the mixture presents the desired consistency.

### Embodiments of the invention

To verify the results obtained with this bio-based binder, as well as comparing it with a standard binder used in orthotics and orthopaedic devices, several tests were performed, and their results are shown in Table 2.

This invention is further illustrated by the following nonlimiting examples.

Example 1 shows the formulation and initial degradation temperature of a standard natural binder used in ecological products.

Example 2 presents the formulation and initial degradation temperature of the bio-based binder of the present invention.

**TABLE 2**

| | Example 1 | Example 2 |
|---|---|---|
| Hydrocolloid Component^{A} | - | 17% |
| Hydrocolloid Component^{B} | 43,75% | |
| Water | - | 43% |
| Alcohol Component^{C} | - | 26% |
| Polyphenolic Compound^{D} | - | 7% |
| Iron Compound^{E} | - | 4% |
| Kaolin | - | 3% |
| Vegetable hydrolysed^{F} | 37,5% | - |
| Alkaline earth metal oxide^{H} | 18,75% | - |
| Initial degradation temperature (°C)^{I} | > 240 °C | 75 - 80 °C |
| Processing temperature (°C) | 230 - 240 °C | 70 - 100 °C |

Caption:
A - Hydrocolloid component: vegetal gelatine
B- Hydrocolloid component: soybean flour
C - Alcohol component: glycerol
D - Polyphenolic compound: tannic acid
E - Iron compound: iron chloride
F - Vegetable hydrolysed: sugarcane bagasse
H - Alkaline earth metal oxide: magnesium oxide
I - Test method: The equipment TA instruments, model Q500 was used to perform the thermogravimetric analysis of different samples in order to understand their thermal stability. A small sample from the material was chosen and used in the analysis. For this, a small sample was placed inside a crucible and heated from 25 °C to 600 °C at a heating rate of 10 °C/ minute. An inert atmosphere was maintained by providing a flow of nitrogen gas at a 60 ml/minute rate. Initial degradation temperature, maximum degradation percentage and weight loss were obtained by graphical means.

The individual components of the formulation normally show a thermogravimetric degradation profile at temperatures above 75-85 °C. Normally, water indicates the start of degradation at 100 °C, where the water present in the binder of the present invention evaporates. Therefore, it was not expected that the binder of the present invention would start to degrade at these temperatures. This could be an advantage, because it makes it possible to process the binder at lower temperatures, without so many economic costs and without the possibility of serious burns. At the same time, the patient or user who uses the binder of the present invention together with an orthotic device will have an internal temperature of around 37 °C, which is not enough to compromise the applicability and use of the binder.

### Bibliographic references:

1. Debnath, S. et al. A Review on Natural Binders used in Pharmacy. Asian Jour. Pharmac. Rese. 9, 55 (2019).
2. Pizzi, A., Papadopoulos, A. N. & Policardi, F. Wood Composites and Their Polymer Binders. Polymers 12, 1115 (2020).
3. Terekhov, I. V. & Chistyakov, E. M. Binders Used for the Manufacturing of Composite Materials by Liquid Composite Molding. Polymers 14, 87 (2021).
4. Zuber, S. H., Hashikin, N. Ab. A., Yusof, M. F. M., Aziz, M. Z. A. & Hashim, R. Influence of Different Percentages of Binders on the Physico-Mechanical Properties of Rhizophora spp. Particleboard as Natural-Based Tissue-Equivalent Phantom for Radiation Dosimetry Applications. Polymers 13, 1868 (2021).
5. Dhawale, P. V. et al. Tannin as a renewable raw material for adhesive applications: a review. Mater. Adv. 3, 3365-3388 (2022).
6. Ekanayake, C., Gamage, J. C. P. H., Mendis, P. & Weerasinghe, P. Revolution in orthopedic immobilization materials: A comprehensive review. Heliyon 9, e13640 (2023).

## Claims

1. Bio-based binder formulation applied in orthotics and orthopaedic devices **characterized in that** it comprises between 15% and 20% by mass of a hydrocolloid component, between 30% and 48% by mass of water, between 10% and 30% by mass of an alcohol component, between 5% and 10% by mass of a polyphenolic compound, between 1% and 5% by mass of iron compounds and between 1% and 3% by mass of kaolin.

2. Bio-based binder formulation according to the previous claim, wherein the hydrocolloid component is selected from: agar agar, vegetal gelatine, pectin, alginate, cellulose, cellulose derivates, xanthan gum, carrageenan and gelatine.

3. Bio-based binder formulation according to any one previous claim, wherein the alcohol component is selected from:
polyhydric alcohol and glycerol.

4. Bio-based binder formulation according to any one of the previous claims, wherein the polyphenolic compound is tannic acid.

5. Bio-based binder formulation according to any one of the previous claims, wherein iron compounds is selected from: iron chloride (III), iron sulphate and iron oxide (III).

6. Process for producing the bio-based binder formulation claimed in the preceding claims, **characterized in that** it comprises the following steps:
a) add the dry components: hydrocolloid component, polyphenolic compound, iron compounds and kaolin;
b) heat the mixture obtained in the previous step to between 70 and 100°C and allow the mixture to dissolve by stirring
c) mix the water with the alcohol component;
d) add the water and alcohol component to the mixture obtained in step b) and mix all the components so that the mixture is homogeneous;
e) the mixture obtained in the previous step will change colour, indicating that the reaction is taking place (to a greenish colour and then to a black material);
f) the mixture obtained in the previous step separates into two phases: a liquid phase and a gel-like material;
g) the liquid phase is discarded;
h) a gel-like material is cooled and allowed to dry at room temperature for at least 24 hours.

7. Process according to the preceding claim, wherein when the selected iron compound is iron oxide (III), additional heating-cooling cycles are carried out, comprising the following steps:
i) when gel-like material obtained in step h) has cooled, it is heated for between 10 and 15 minutes until it reaches the temperature of at least 70 °C;
j) a heated mixture is obtained and allowed to cool again for 30 and 45 minutes;
k) repeat the heating-cooling steps: steps i) and j).

8. Use of the bio-based binder formulation claimed in any claims 1 to 5 in orthotics and orthopaedic devices.
